Europäisches Patentamt

**European Patent Office**

**Office européen des brevets**

(19)

(11) Numéro de publication : **0 138 648**
**B1**

(12)

# FASCICULE DE BREVET EUROPÉEN

(45) Date de publication du fascicule du brevet :
**13.04.88**

(51) Int. Cl.⁴ : **A 61 F  2/48**

(21) Numéro de dépôt : **84401761.6**

(22) Date de dépôt : **05.09.84**

(54) **Dispositif de commande du débit d'un fluide et organe prothétique équipe de ce dispositif.**

(30) Priorité : **14.09.83 FR 8314607**

(43) Date de publication de la demande :
**24.04.85 Bulletin 85/17**

(45) Mention de la délivrance du brevet :
**13.04.88 Bulletin 88/15**

(84) Etats contractants désignés :
**AT BE CH DE FR GB IT LI LU NL SE**

(56) Documents cités :
**FR-A- 2 373 272**
**US-A- 3 503 400**
**US-A- 3 841 304**
**US-A- 3 854 469**

(73) Titulaire : **CENTRE NATIONAL DE LA RECHERCHE
SCIENTIFIQUE (CNRS)**
**15, Quai Anatole France**
**F-75700 Paris (FR)**

(72) Inventeur : **Rey, Pierre**
**18 rue Aristide Briand**
**F-77400 Thorigny (FR)**
Inventeur : **Leandri, Jacqueline née Cesari**
**50 Avenue de Clichy**
**F-75018 Paris (FR)**
Inventeur : **Abbou, Clément**
**43 Avenue de la Dame Blanche**
**F-94120 Fontenay-sous-Bois (FR)**

(74) Mandataire : **Orès, Bernard et al**
**Cabinet ORES 6, Avenue de Messine**
**F-75008 Paris (FR)**

Jouve, 18, rue St-Denis, 75001 Paris, France

EP 0 138 648 B1

## Description

La présente invention est relative à un dispositif de commande du débit d'un fluide, notamment d'un fluide biologique et en particulier du débit urinaire, ainsi qu'à un organe prothétique équipé de ce dispositif.

Le Brevet français FR-A-2 251 302 décrit une prothèse sphinctérienne, implantable en position endo-urétrale ou ano-rectale notamment, qui est munie d'un mécanisme d'activation externe du type électro-magnétique ; de façon plus précise, le dispositif de réglage, qui est destiné à remplacer un sphincter détruit ou déficient, est constitué par une portion de tube logée dans le conduit naturel intéressé et par un ballonnet gonflable inséré à l'intérieur de cette portion de tube, en sorte que la lumière de ce dernier est obstruée à l'état complètement gonflé, tandis que l'ouverture de cette lumière est obtenue par déflation du ballonnet.

L'obturation et l'ouverture de cette portion de tube, à savoir l'inflation et la déflation dudit ballonnet, sont obtenues par l'intermédiaire d'un fluide qui remplit le ballonnet et d'une chambre d'expansion délimitée par un soufflet métallique et communiquant avec le ballonnet par un conduit souple, qui est de préférence entouré par une collerette évasée d'étanchéité près de son point d'implantation dans la portion de tube : les mouvements de ce fluide sont commandés par un dispositif électro-magnétique agissant à travers la peau.

Toutefois, la manipulation de ce dispositif électromagnétique de commande est peu aisée et sa conception relativement lourde.

On connaît également un dispositif de sphincter artificiel à commande hydraulique qui est décrit par le Brevet français FR-A-2 373 272 et qui comprend :

- un collier de serrage de l'urètre par gonflage sous l'action du liquide sous pression,

- au moins un réservoir déformable de gonflage de ce collier déformable,

- un système de clapet disposé entre ce réservoir de gonflage et le collier de serrage, et

- des moyens de dégonflage de ce collier, pouvant éventuellement comprendre un réservoir déformable auxiliaire de dégonflage.

Ce dispositif comporte :

- un clapet unique appliqué contre un siège ménagé dans une cloison prévue à l'intérieur d'un boîtier et séparant une première chambre — délimitée en coopération avec un premier fond du boîtier, notamment celui qui se trouve du côté du collier de serrage — d'une deuxième chambre communiquant avec ledit réservoir déformable de gonflage de ce collier, et

- des moyens de commande du dégonflage dudit collier.

Selon un mode de réalisation de ce dispositif sphinctérien, qui correspond à l'absence dudit réservoir auxiliaire déformable de dégonflage :

- ladite cloison pourvue du siège de clapet et les parois dudit boîtier sont déformables, de manière à constituer elles-mêmes lesdits moyens de commande du dégonflage du collier de serrage, dans ce cas le clapet unique étant constitué par une languette élastique, donc déformable, fixée à cette cloison par une de ses extrémités,

- le boîtier déformable et ledit réservoir déformable de gonflage peuvent être montés sur un support rigide.

Ledit boîtier comporte deux tubulures reliées auxdits premier et deuxième fonds : de façon plus précise, la première tubulure relie ladite première chambre audit collier de serrage, tandis que la deuxième tubulure relie la deuxième chambre audit réservoir déformable de gonflage.

Or, l'inconvénient principal de ce deuxième Brevet français est constitué par la solidarisation de ladite languette élastique à ladite cloison et par le fait que la séparation à partir de l'application normale contre son siège, lors du gonflage du collier par déformation dudit réservoir, demande l'exercice d'une pression relativement élevée, ce qui justifie l'emploi dudit support rigide.

En outre, le Brevet américain US-A-3 854 469 décrit un dispositif de contrôle du débit d'un fluide à travers un conduit anatomique traversé par un fluide physiologique, tel que de l'urine à travers l'urètre. Ce dispositif comprend :

- un premier moyen de gonflage constitué par une première chambre souple contenant, en condition de repos, une quantité de fluide suffisante pour arrêter le débit de fluide dans ledit conduit anatomique lorsqu'elle est appliquée contre la paroi externe de ce conduit,

- un moyen de soupape pourvu d'un passage de fluide relativement étroit qui est sensible à la différence de pression hydrostatique existant entre les extrémités de ce passage, de même qu'à la pression obtenue par déformation manuelle de ladite première chambre ou par déformation de :

- un deuxième moyen de gonflage constitué par une deuxième chambre souple, qui est reliée à la première chambre par ledit moyen de soupape et qui est destinée à recevoir le fluide initialement contenu dans cette première chambre sous l'action d'une pression appliquée par déformation manuelle de cette dernière, le débit d'écoulement du fluide de ladite première chambre vers ladite deuxième chambre étant relativement élevé tandis que le débit dans le sens inverse (à savoir de cette deuxième chambre vers la première), sous l'action de la différence de pression hydrostatique existant entre la première et la deuxième chambres, est relativement faible jusqu'à s'annuler lorsque s'établit l'équilibre des pressions entre les deux chambres, à l'annulation du débit correspondant la fermeture dudit passage de fluide (le temps d'écoulement sous l'action de ladite différence de pression hydrostatique est fonction des dimensions — longueur et diamètre — de ce passage : ce temps peut être diminué en exerçant la pression obtenue par déformation manuelle aussi de la deuxième chambre).

En outre, ce Brevet américain précise que :

- la première chambre est maintenue appliquée contre la paroi externe du conduit dont on veut contrôler le débit à l'aide d'un ruban,

- la deuxième chambre est pourvue d'un bouchon en caoutchouc naturel permettant d'ajouter, le cas échéant, du fluide par piqûre hypodermique, et

- le moyen de soupape comprend un corps élastique, notamment en élastomère de silicone qui est dilatable sous l'action des manipulations desdites chambres visant à établir un débit d'écoulement relativement élevé entre ces chambres.

L'inconvénient principal de ce Brevet américain est qu'il est sensible également à la différence de pression hydrostatique existant entre lesdites deux chambres déformables, ce qui n'est pas toujours souhaitable dans ce type d'applications.

De plus, son implantation sous-cutanée, de même que celle dudit deuxième Brevet Français, est relativement compliquée.

Il existe également une prothèse urétrale qui est mise en place par voie endo-canalaire, et qui est donc interchangeable (cf. l'article des Annales d'Urologie, 1982, 16, n° 5 pages 285-288).

Cette prothèse urétrale est constituée par un tube de 15 cm environ de longueur et d'un diamètre variable, réalisé en élastomère de silicone.

Ce tube présente :

- des surfaces anti-adhérentes obtenues par moulage,

- deux orifices de drainage situés à 1 cm de son extrémité proximale, et

- une double pluralité d'ergots prévenant son déplacement axial, sans toutefois en empêcher l'extraction manuelle, qui sont ménagés sur la partie moyenne extérieure du tube et qui sont dirigés vers le haut et vers le bas, respectivement.

L'avantage incontesté de cette prothèse urétrale est de pouvoir être changée, à la demande, sans chirurgie ; toutefois, son emploi est limité aux urétrites radiques et aux sténoses multiples récidivantes chez l'homme seulement et n'intéresse pas les incontinences.

On connaît également le Brevet US-A-3 503 400 qui décrit un dispositif de contrôle de l'écoulement d'un fluide biologique destiné à être implanté dans l'organisme d'un patient, notamment affecté d'une incontinence urinaire, lequel dispositif comporte en combinaison :

- une valve de contrôle de l'écoulement urinaire disposée au fond du canal urétral,

- un moyen d'actionnement par l'extérieur de l'organisme de cette valve, constitué par un fil flexible (enfilé dans un conduit) pouvant être actionné par le patient,

- un moyen d'attache tubulaire entourant le fil d'actionnement et s'étendant sur toute la longueur de l'urètre, à partir de l'emplacement de la valve jusqu'à l'extérieur, pour protéger les tissus de l'organisme contre le contact direct avec le fil d'actionnement.

Ce document précise, en outre, que le dispositif de contrôle de l'écoulement urinaire comporte un moyen destiné à assurer l'étanchéité de l'extrémité proximale du dispositif par rapport à la vessie du patient.

La présente invention a en conséquence pour but de pourvoir à un dispositif de commande du débit d'un fluide, notamment d'un fluide biologique et en particulier du débit urinaire, à travers un conduit anatomique éventuellement implantable en position sous-cutanée, qui répond mieux aux nécessités de la pratique que les dispositifs visant au même but antérieurement connus, notamment :

- en ce que sa manipulation est très simple,

- en ce que son éventuelle implantation en position sous-cutanée est aisée pour le chirurgien et représente un minimum de risques opératoires pour le malade, ainsi qu'un minimum d'altérations pour ce dernier en ce qui concerne son anatomie et ses fonctions organiques,

- en ce que l'effort nécessaire pour s'en servir est minimal.

La présente invention a également pour but de pourvoir à des organes prothétiques interchangeables, notamment à des prothèses urétrales qui répondent mieux aux nécessités de la pratique que les organes prothétiques visant au même but antérieurement connus, notamment en ce qu'ils sont équipés d'un dispositif de commande du débit urinaire conforme aux dispositions de la présente invention, en sorte que :

- leur utilisation peut s'étendre à un grand nombre de cas pathologiques ayant trait aux incontinences urinaires, y compris les incontinences d'origine nerveuse et les incontinences postopératoires (après les interventions chirurgicales sur la prostate chez l'homme notamment),

- ils se prêtent, par ladite coopération avec le dispositif de commande conforme à l'invention, à la réalisation de prothèses urétrales à utilisation non seulement masculine, mais également féminine,

- l'ensemble organe prothétique/dispositif de commande du débit peut être mis en place sans chirurgie dans le cas où le dispositif de commande selon l'invention est incorporé dans les parois de l'organe prothétique, chez l'homme notamment, ou dans le cas où ledit dispositif de commande est en position urétro-vaginale, éventuellement dépassant du vagin, chez la femme notamment.

La présente invention a pour objet un dispositif de commande du débit d'un fluide, notamment d'un fluide biologique et en particulier du débit urinaire, du type comportant :

- soit deux ballonnets activateurs souples reliés par un tube de liaison, notamment en élastomère de silicone, dont le premier est destiné à commander, par dégonflage par déformation manuelle, le rétablissement du débit dudit fluide biologique, tandis que le deuxième ballonnet activateur est destiné à commander, également par dégonflage par déformation manuelle, l'annulation de ce débit ;

- soit lesdits deux ballonnets activateurs souples reliés par ledit tube de liaison, et un troisième ballonnet souple, dont le dégonflage et le gonflage, commandés par déformation manuelle desdits premier et deuxième ballonnets activateurs, respectivement, permettent de contrôler le débit dudit fluide biologique, notamment par insertion de ce troisième ballonnet dans la lumière du conduit dont on désire contrôler le débit, ledit troisième ballonnet étant relié notamment au premier ballonnet activateur par une première tubulure de liaison de longueur appropriée,

- et un système de soupape du type sensible uniquement à la pression obtenue par déformation manuelle de l'un desdits premier et deuxième ballonnets activateurs,

lequel dispositif est caractérisé en ce que ledit système de soupape comporte une tige qui, en condition de repos, est enserrée de façon étanche, par tout moyen de serrage approprié, à l'intérieur dudit tube de liaison, qui est réalisé en une matière plastique souple présentant un retrait à la polymérisation, et en ce qu'un moyen est également prévu qui est apte à empêcher le déplacement axial de cette tige de soupape le long du tube de liaison sous l'effet de ladite pression.

Selon un mode de réalisation avantageux du dispositif conforme à l'invention, le moyen de serrage de la tige est constitué par un deuxième tube disposé autour du premier tube.

Selon un autre mode de réalisation avantageux du dispositif conforme à l'invention, ledit moyen de serrage est constitué par au moins un joint torique.

Selon un autre mode de réalisation avantageux du dispositif conforme à l'invention, ledit moyen de serrage est constitué par une bague.

Selon encore un autre mode de réalisation avantageux, ledit moyen de serrage est constitué par un ressort.

Selon un mode de réalisation préféré du dispositif conforme à l'invention, ledit moyen de serrage est constitué par la paroi même dudit tube.

Selon un mode de réalisation avantageux du dispositif conforme à l'invention, le moyen empêchant tout déplacement axial de la tige est un moyen pris dans le groupe qui comprend : un disque, ou un croisillon, ou un ergot, ou un élément en forme de « queue de cochon », ou encore une sphère ou bille, donnant à la tige une configuration en haltère et dont est pourvue chaque extrémité de cette tige, les moyens dont sont pourvues les deux extrémités de la tige pouvant être identiques ou différents.

Selon un autre mode de réalisation avantageux du dispositif conforme à l'invention, le moyen empêchant tout déplacement axial de la tige est constitué par un rétrécissement du diamètre dudit tube de liaison au niveau des extrémités de la tige, obtenu par moulage.

Selon une variante avantageuse de ce mode de réalisation, deux tubulures de liaison auxiliaires, de diamètre inférieur au diamètre dudit tube contenant la tige et réalisées en le même matériau que lesdits premier et deuxième ballonnets activateurs souples, relient ce tube à ces ballonnets, interdisant ainsi tout déplacement axial de la tige qu'il contient.

Selon encore un autre mode de réalisation avantageux du dispositif conforme à l'invention, ladite première tubulure de liaison, reliant ledit troisième ballonnet à l'un desdits premier et deuxième ballonnets activateurs, comporte une coupure intermédiaire et un dispositif de jonction rapide étanche, notamment à clips, reconstituant sa continuité physique dans la zone de ladite coupure.

Outre les dispositions qui précèdent, l'invention comprend encore d'autres dispositions, qui ressortiront de la description qui va suivre.

L'invention sera mieux comprise à l'aide du complément de description qui va suivre, qui se réfère aux dessins annexés dans lesquels :

- les figures 1a et 2 représentent schématiquement deux modes de réalisation différents du dispositif de commande du débit d'un fluide, conformes à la présente invention ;

- la figure 1b se réfère à une variante du dispositif représenté à la figure 1a ;

- la figure 1c montre le détail seulement d'une autre variante du dispositif de figure 1a ;

- la figure 3 est la représentation schématique de l'application du mode de réalisation du dispositif conforme à la figure 1b, à une prothèse urétrale interchangeable à utilisation masculine,

- la figure 4 est la représentation schématique de l'application du mode de réalisation du dispositif conforme à la figure 2, à une prothèse urétrale interchangeable à utilisation féminine, et

- la figure 5 illustre en section les détails correspondant à un dispositif de jonction rapide étanche j destiné à reconstituer la continuité physique dans la tubulure t de la figure 4.

Il doit être bien entendu, toutefois, que ces dessins et les parties descriptives correspondantes, sont donnés uniquement à titre d'illustration de l'objet de l'invention, dont ils ne constituent en aucune manière une limitation.

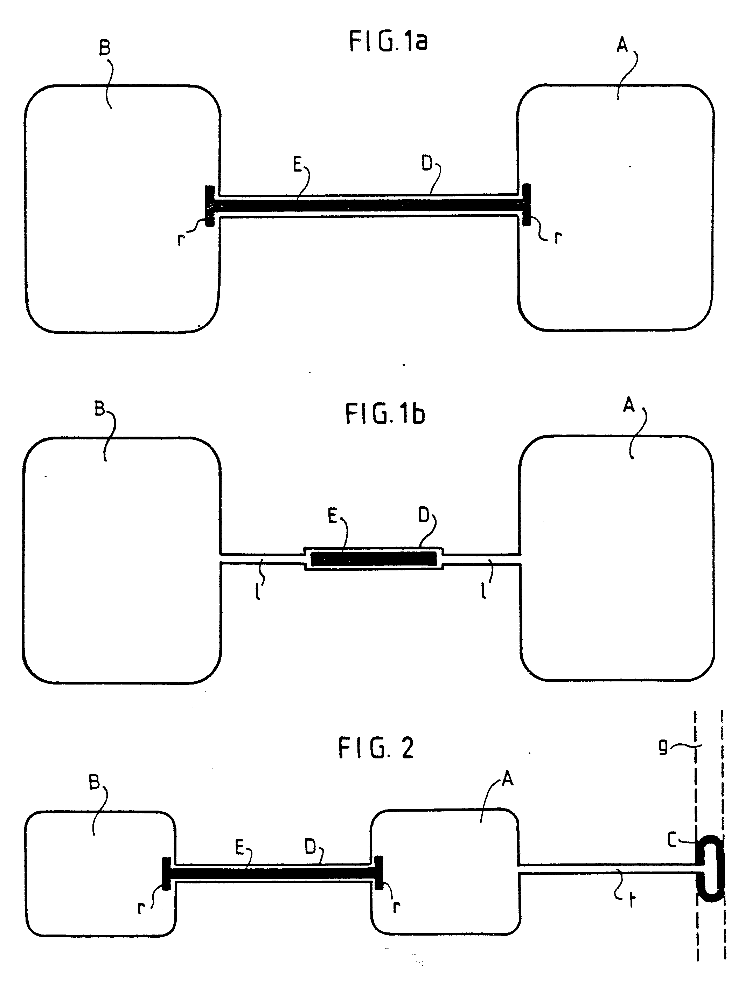

La figure 1a se réfère à un mode de réalisation préféré du dispositif de commande du débit d'un fluide, notamment d'un fluide biologique et en particulier du débit urinaire, conforme à la présente invention.

Ce mode de réalisation préféré est constitué par un système fermé rempli d'un fluide et comportant deux ballonnets activateurs A et B souples reliés par un tube D dont la paroi serre directement et de façon étanche une tige E insérée à l'intérieur dudit tube.

Toutefois, d'autres modes de réalisation sont envisageables dans lesquels le serrage de ladite tige E se fait par tous moyens assurant également la séparation étanche entre les ballonnets activateurs souples, en condition de repos.

Le déplacement axial de la tige E dans le tube D est empêché en équipant chacune des extrémités de cette tige E d'un disque r, par exemple, comme indiqué à la figure 1a.

A cet effet, il est possible de donner aux extrémités de ladite tige E une configuration différente, notamment celle d'un croisillon, d'un ergot, d'une « queue de cochon " ou d'une sphère (ou bille).

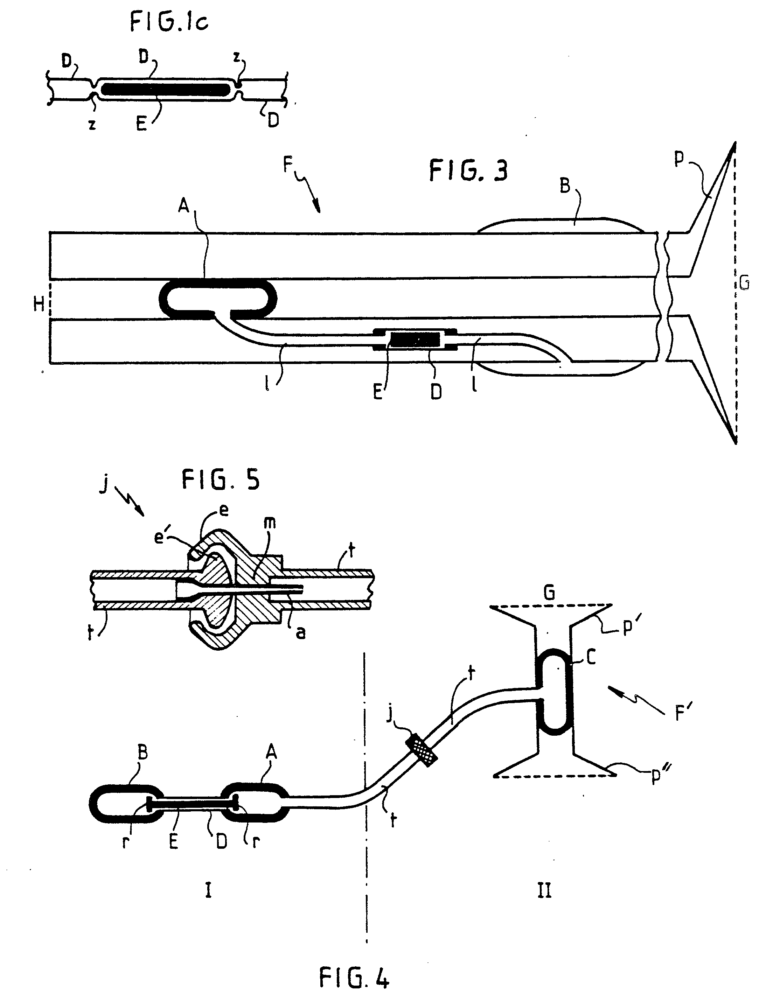

Le même résultat peut être obtenu, en outre :
- en reliant le tube D à chacun des deux ballonnets activateurs A et B par une tubulure 1 dont le diamètre est inférieur au diamètre du tube D, cette variante étant représentée à la figure 1b, ou encore
- en réalisant par moulage un rétrécissement z (cf. la figure 1c) sur ledit tube de liaison D, au niveau des extrémités de la tige E.

Etant donné que les deux ballonnets activateurs et le tube D sont réalisés en toutes matières plastiques souples qui présentent un retrait à la polymérisation lors du durcissement, en silicone de préférence ou en polyuréthane, notamment, le tube D serre de façon étanche la tige E qu'il contient, assurant ainsi la séparation étanche des deux ballonnets A et B, dont le volume est maximum en position de repos.

Le principe de fonctionnement du dispositif de commande du débit repose sur ladite étanchéité entre deux ballonnets activateurs A et B, ainsi que sur l'élasticité de la paroi du tube D et sur la tendance des ballonnets A et B à reprendre leur forme d'origine dès qu'ils ne sont pas sollicités.

De façon plus précise, une augmentation de pression par exemple dans le ballonnet A, obtenue par appui manuel ou digital, provoque l'écartement de la paroi du tube D de la surface de la tige E et, par conséquent, le passage du fluide comprimé contenu dans le ballonnet A vers le ballonnet B, où le fluide est stocké.

Au moment du relâchement de l'appui sur le ballonnet A, le fluide stocké en B ne revient pas spontanément vers A, du fait que, si ce dernier tend à reprendre sa forme initiale, le tube D s'oppose toutefois à ce mouvement en se resserrant de façon étanche sur la tige E, ce qui provoque une dépression dans le ballonnet A et donc une aspiration de fluide du ballonnet A vers le ballonnet B, à savoir la déflation du premier ballonnet A.

Il va de soi qu'une augmentation de pression dans le ballonnet B provoque le phénomène inverse et le transfert du fluide de B vers A, et donc la déflation du ballonnet B et l'inflation du ballonnet A.

Sur la base de ce qui précède, on comprend très bien que si, par exemple, le ballonnet activateur A est inséré dans un conduit, il est possible d'ouvrir et d'obturer, respectivement, la lumière de ce conduit en procédant de la manière décrite plus haut, à savoir en comprimant alternativement les deux ballonnets activateurs A et B : Il va de soi que la forme d'un des deux ballonnets (dans ce cas celle du ballonnet B) peut être a priori quelconque, par exemple sphérique, annulaire ou autre, mais de toutes façons telle qu'elle puisse s'adapter aux différentes applications dont est susceptible le dispositif de commande conforme à l'invention, tandis que la forme de l'autre ballonnet, (dans ce cas le ballonnet A) doit pouvoir s'adapter, à l'état de repos, à la forme du conduit qu'il doit obturer, pour pouvoir remplir le rôle de ballonnet à la fois activateur et obturateur du conduit dont le débit doit être commandé.

Une application très intéressante de cette possibilité de commande est indiquée à la figure 3, dans laquelle F représente une prothèse urétrale interchangeable à utilisation masculine, du type comportant un tube en élastomère de silicone, de préférence, pourvu d'ergots (non représentés) qui préviennent son déplacement axial ; cette prothèse est équipée d'un dispositif de commande du débit urinaire conforme au premier mode de réalisation du dispositif selon l'invention, constitué par la représentation de la figure 1b.

Un tube D de serrage d'une tige E est inséré dans la paroi de la prothèse urétrale interchangeable F ; un ballonnet A est inséré à l'intérieur du conduit de la prothèse F, en amont du méat urinaire H, tandis qu'un ballonnet B, annulaire, est placé extérieurement au conduit de cette prothèse F, autour de son extrémité vésicale qui est avantageusement pourvue d'une collerette évasée p, à l'extrémité proximale, assurant l'étanchéité par rapport à la vessie G, chacun des deux ballonnets A et B étant relié au tube D par une tubulure 1 insérée elle aussi dans la paroi de la prothèse F.

Le fonctionnement de l'urètre prothétique interchangeable F, mis en place dans la verge, est le suivant :
- une pression de la verge dans la zone du ballonnet A provoque le transfert du fluide qui y est contenu, vers le ballonnet B ;
- le relâchement de la pression du ballonnet A détermine l'ouverture de la lumière de l'urètre prothétique F ; cette ouverture persiste, ce qui permet la miction ;
- ensuite, dès la fin de la miction, une pression de la verge dans la zone du ballonnet B provoque le refoulement du fluide qui y est contenu, vers le ballonnet A ;
- le relâchement de la pression du ballonnet B détermine la fermeture de la lumière de la prothèse urétrale F par inflation complète du ballonnet A.

La figure 2 se réfère à un deuxième mode de réalisation du dispositif de commande du débit d'un fluide, conforme à la présente invention.

Ce deuxième mode de réalisation est constitué par un système fermé rempli d'un fluide et comportant, comme le dispositif de commande conforme au premier mode de réalisation représenté à la figure 1a, deux ballonnets activateurs A et B reliés directement par un tube D qui serre une tige E en forme d'haltère ; en outre, ce deuxième mode de réalisation comporte un troisième ballonnet C relié au ballonnet A par une tubulure de liaison t et destiné à obturer la lumière d'un conduit g, d'un sphincter artificiel notamment.

Le principe de fonctionnement dans ce deuxième cas est le même que celui du dispositif représenté à la figure 1a et de sa variante représentée à la figure 1b : un appui sur le premier ballonnet activateur A provoque une augmentation de la pression à l'intérieur de ce dernier, ainsi qu'à l'intérieur du ballonnet obturateur C — du fait de leur communication par l'intermédiaire d'une troisième tubulure de liaison t — et, par conséquent, l'écartement de la paroi du tube D de la surface de la tige E, et le passage du fluide comprimé contenu dans les ballonnets A et C vers le ballonnet B, où le fluide est stocké.

Au moment du relâchement de l'appui sur le ballonnet A, la dépression qui vient se créer dans ce dernier (comme précisé plus haut) provoque une aspiration du fluide contenu dans le ballonnet C vers le premier ballonnet activateur A et donc la déflation du ballonnet obturateur C et l'ouverture du conduit g.

Inversement, un simple appui sur le deuxième ballonnet activateur B a pour résultat une augmentation de la pression dans ce dernier, provoquant le transfert du fluide de B vers A et C, et donc l'inflation du ballonnet obturateur C et la fermeture du conduit g.

En ce qui concerne la tubulure de liaison t entre les ballonnets A et C, celle-ci peut avantageusement comporter une coupure intermédiaire et un dispositif de jonction rapide étanche j (cf. la figure 4) reconstituant sa continuité physique dans la zone de cette coupure.

Ce dispositif j de jonction rapide étanche est avantageusement du type à clips, comme représenté à la figure 5. L'extrémité du tronçon de la tubulure t, qui se trouve du côté des ballonnets activateurs A et B, est pourvue d'un renflement e coopérant avec une aiguille a qui le traverse et qui est solidarisée avec ce renflement et la partie terminale dudit tronçon de la tubulure t. En ce qui concerne l'extrémité de l'autre tronçon de la tubulure t, celle-ci est pourvue d'une coiffe e', s'adaptant par encliquetage sur le renflement e, et un manchon central m, qui est percé axialement pour recevoir, lors de l'encliquetage, ladite aiguille a, reconstituant ainsi de façon étanche la nécessaire continuité physique entre les deux tronçons de ladite tubulure t.

Une autre application très intéressante de la possibilité de commande de l'ouverture d'un conduit, fournie par le dispositif selon l'invention et analogue à celle représentée à la figure 3, est indiquée à la figure 4, dans laquelle F' représente une prothèse urétrale interchangeable à utilisation féminine, qui est également du type comportant un tube en élastomère de silicone, de préférence, pourvu d'ergots (non représentés), sur sa surface extérieure, prévenant son déplacement axial.

Cette prothèse F' présente, de même que la prothèse à utilisation masculine F, une collerette p' à son extrémité proximale, pour assurer l'étanchéité par rapport à la vessie G ; à cet égard, il y a lieu de souligner que l'étanchéité peut être obtenue (dans les deux cas d'utilisation de la prothèse urétrale), selon une variante avantageuse, également par un ballonnet annulaire (non représenté) entourant l'extrémité proximale du tube prothétique qui est introduit dans la vessie G à la façon d'une sonde de Foley.

La prothèse F' présente aussi une autre collerette p'' à son extrémité distale, tenant compte des différences anatomiques liées à l'utilisation féminine de la prothèse uréthrale F', qui coopère également dans ce cas avec un dispositif de commande du débit urinaire, conforme au deuxième mode de réalisation du dispositif selon l'invention, représenté à la figure 2, lequel dispositif de commande est implantable en position sous-cutanée, en sorte que l'ensemble prothèse urétrale/dispositif de commande constitue un modèle partiellement interchangeable, en ce qui concerne l'urètre prothétique F'.

On retrouve à la figure 4, dans la zone I correspondant à l'implantation sous-cutanée, le dispositif de commande de la figure 2, tandis que la zone II, qui est séparée de la zone I par une ligne discontinue, correspond à la prothèse urétrale F' en position urétro-vaginale : un dispositif j de jonction rapide étanche, notamment à clips, du type déjà décrit, est prévu sur la portion urétrovaginale de la tubulure de liaison t, entre le ballonnet activateur A et le ballonnet obturateur C, de façon à faciliter l'interchangeabilité de la prothèse urétrale F' sans toucher au dispositif activateur implanté. La zone d'implantation I est choisie, comme il est évident, de manière que la personne intéressée ne puisse appuyer dessus de façon accidentelle.

Alternativement, on peut utiliser un ensemble prothèse urétrale/dispositif de commande complètement interchangeable, comme dans le cas d'utilisation masculine (cf. la figure 3), à condition que l'ensemble prothétique contenant le mécanisme d'ouverture et de fermeture soit en position vaginale éventuellement dépassant du vagin.

Par contre, dans certains cas, il peut s'avérer nécessaire de réaliser l'implantation en position sous-cutanée du dispositif de commande du débit urinaire coopérant avec une prothèse urétrale F à utilisation masculine, selon le principe illustré lors de la description de la prothèse urétrale F' à utilisation féminine.

Il va de soi que le dispositif de commande est également applicable à la prothèse sphinctérienne implantable, décrite dans le Brevet FR-A-2 251 302, ainsi qu'à tout autre organe prothétique approprié.

En ce qui concerne l'obtention d'un serrage étanche, en condition de repos, entre le tube D et la tige E qu'il contient, cela est obtenu, pour chaque mode de réalisation (cf. les figures 1a, 1b, et 2, ainsi que les applications de ces modes de réalisation représentées aux figures 3 et 4), grâce au procédé de moulage d'un élastomère de silicone de préférence, ou de polyuréthane notamment ou, en général, de toute matière plastique souple présentant un retrait à la polymérisation lors du durcissement : cette matière plastique est moulée autour de la tige E qui est mise en place au préalable dans un moule et qui est enveloppée, après le moulage, par le tube de serrage D ainsi moulé.

## Revendications

1. Dispositif de commande du débit d'un fluide, notamment d'un fluide biologique à travers un conduit anatomique et en particulier du débit urinaire, du type comportant :

- soit deux ballonnets activateurs souples (A, B) reliés par un tube de liaison (D), notamment en élastomère de silicone, dont le premier (A) est destiné à commander, par dégonflage par déformation manuelle, le rétablissement du débit dudit fluide biologique, tandis que le deuxième ballonnet activateur (B) est destiné à commander, également par dégonflage par déformation manuelle, l'annulation de ce débit ;

- soit lesdits deux ballonnets activateurs souples (A, B) reliés par ledit tube de liaison (D), et un troisième ballonnet souple (C), dont le dégonflage et le gonflage, commandés par déformation manuelle desdits premier et deuxième ballonnets activateurs (A, B), respectivement, permettent de contrôler le débit dudit fluide biologique, notamment par insertion de ce troisième ballonnet (C) dans la lumière du conduit dont on désire contrôler le débit, ledit troisième ballonnet (C) étant relié notamment au premier ballonnet activateur (A) par une première tubulure de liaison (t) de longueur appropriée,

- et un système de soupape du type sensible uniquement à la pression obtenue par déformation manuelle de l'un desdits premier et deuxième ballonnets activateurs (A, B), lequel dispositif est caractérisé en ce que ledit système de soupape comporte une tige (E) qui, en condition de repos, est enserrée de façon étanche, par tout moyen de serrage approprié, à l'intérieur dudit tube de liaison (D), qui est réalisé en une matière plastique souple présentant un retrait à la polymérisation, et en ce qu'un moyen est également prévu qui est apte à empêcher le déplacement axial de cette tige de soupape (E) le long du tube de liaison (D) sous l'effet de ladite pression.

2. Dispositif selon la revendication 1, caractérisé en ce que le moyen de serrage de la tige (E) est constitué par un deuxième tube disposé autour dudit tube de liaison (D).

3. Dispositif selon la revendication 1, caractérisé en ce que ledit moyen de serrage est constitué par une bague disposée autour dudit tube de liaison (D).

4. Dispositif selon la revendication 1, caractérisé en ce que ledit moyen de serrage est constitué par un ressort disposé autour dudit tube de liaison (D).

5. Dispositif selon la revendication 1, caractérisé en ce que ledit moyen de serrage est constitué par la paroi même dudit tube de liaison (D).

6. Dispositif selon la revendication 1, caractérisé en ce que le moyen empêchant tout déplacement axial de la tige (E) est un moyen pris dans le groupe qui comprend : un disque (r), ou un croisillon, ou un ergot, ou un élément en forme de « queue de cochon », ou encore une sphère ou bille, donnant à la tige (E) une configuration en haltère et dont est pourvue chaque extrémité de cette tige (E), les moyens dont sont pourvues les deux extrémités de la tige pouvant être identiques ou différents.

7. Dispositif selon la revendication 1, caractérisé en ce que le moyen empêchant tout déplacement axial de la tige (E) est constitué par un rétrécissement (z) du diamètre dudit tube de liaison (D) au niveau des extrémités de la tige (E), obtenu par moulage.

8. Dispositif selon la revendication 1, caractérisé en ce que deux tubulures de liaison auxiliaires (1), de diamètre inférieur au diamètre dudit tube de liaison (D) contenant la tige (E) et réalisées en le même matériau que lesdits premier et deuxième ballonnets activateurs souples (A et B, respectivement), relient ce tube (D) à ces ballonnets (A et B, respectivement), interdisant ainsi tout déplacement axial de la tige (E) qu'il contient.

9. Dispositif selon la revendication 1, caractérisé en ce que ladite première tubulure de liaison (t), reliant ledit troisième ballonnet (C) à l'un desdits premier et deuxième ballonnets activateurs (A ou B), comporte une coupure intermédiaire et un dispositif (j) de jonction rapide étanche, notamment à clips, reconstituant sa continuité physique dans la zone de ladite coupure.

10. Organe prothétique constitué par une prothèse urétrale interchangeable, comportant un tube en élastomère de silicone, de préférence et un moyen assurant l'étanchéité de l'extrémité proximale de ce tube par rapport à la vessie (G), lequel organe prothétique est caractérisé en ce qu'il est pourvu d'une pluralité d'ergots ménagés sur la surface extérieure dudit tube et prévenant le déplacement axial de ce dernier, certains ergots étant dirigés vers le bas, tandis que d'autres ergots sont dirigés vers le haut, et en ce qu'il comporte un dispositif de commande du débit urinaire, selon l'une quelconque des revendications 1 à 9.

11. Organe prothétique selon la revendication 10, caractérisé en ce que la longueur et le diamètre de son tube sont appropriés à l'utilisation masculine, et en ce que ledit dispositif de commande est conforme aux revendications 1 et 8 ou 7 et est inclus dans la paroi du tube de l'organe prothétique.

12. Organe prothétique selon la revendication 10, caractérisé en ce que la longueur et le diamètre de son tube sont appropriés à l'utilisation masculine, et en ce que ledit dispositif de commande est conforme aux revendications 1, 6 et 9 et est implantable en position sous-cutanée appropriée.

13. Organe prothétique selon la revendication 10, caractérisé en ce que la longueur et le diamètre de son tube sont appropriés à l'utilisation féminine, en ce que ledit tube prothétique est pourvu d'une deuxième collerette (p") à son extrémité distale, et en ce que ledit dispositif de commande est conforme aux revendications 1, 6 et 9 et est implantable en position sous-cutanée appropriée.

14. Organe prothétique selon la revendication 10, caractérisé en ce que la longueur et le diamètre de son tube sont appropriés à l'utilisation féminine, en ce que ledit tube prothétique est pourvu d'une deuxième collerette (p") à son extrémité distale, et en ce que ledit dispositif de commande est conforme aux revendications 1, 6 et 9 et est en position vaginale éventuellement dépassant du vagin.

15. Organe prothétique selon l'une quelconque des revendications 10 à 14, caractérisé en ce que ledit moyen d'étanchéité dont est pourvue l'extrémité proximale dudit tube prothétique est constitué par une collerette (p ou p') assurant l'étanchéité nécessaire par rapport à la vessie (G).

16. Organe prothétique selon l'une quelconque des revendications 10 à 14, caractérisé en ce que ledit moyen d'étanchéité, dont est pourvue l'extrémité proximale dudit tube prothétique, est constitué par un ballonnet annulaire qui l'entoure, assurant ainsi l'étanchéité nécessaire par rapport à la vessie (G), à la façon d'une sonde de Foley notamment.

## Claims

1. Device for controlling the flow of a fluid, particularly a biological fluid through an anatomical duct and in particular the urinary flow, of the type comprising :
- either two flexible activating bags (A, B) connected together by a connection tube (D) made more especially from silicon elastomer, the first (A) of which is intended to control, through deflation by manual deformation, the reestablishment of the flow of said biological fluid, whereas the second activating bag (b) is intended to control, also by deflation through manual deformation, the cancellation of this flow ;
- or said two flexible activating bags (A, B) connected together by said connection tube (t), and a third flexible bag (C), the deflation and inflation of which controlled by manual deformation of said first and second activating bags (A, B), respectively, permit the control of the flow of said biological fluid, more especially by insertion of this third bag (C) in the aperture of the duct whose flow it is desired to control, said third bag (C) being connected more particularly to the first activating bag (A) by a first connection pipe (t) of appropriate length,
- and a valve system of the type sensitive only to the pressure obtained by manual deformation of one of said first and second activating bags (A, B),
which device is characterized in that said valve system comprises a rod (Z) which, in the rest condition, is sealingly enclosed, by any appropriate clamping means, inside said connection tube (D), which is made from a flexible plastic material shrinking on polymerization, and in that a means is also provided which is adapted for preventing the axial movement of this valve rod (E) along the connection tube (D) under the effect of said pressure.

2. Device as claimed in claim 1, characterized in that the means for clamping the rod (E) is formed by a second tube disposed about said connection tube (D).

3. Device as claimed in claim 2, characterized in that said clamping means is formed by a ring disposed about said connection tube (D).

4. Device as claimed in claim 1, characterized in that said clamping means is formed by a spring disposed about said connection tube (D).

5. Device as claimed in claim 1, characterized in that said clamping means is formed by the wall itself of said connection tube (D).

6. Device as claimed in claim 1, characterized in that the means preventing any axial movement of the rod (D) is a means chosen from the group which includes : a disk (R), or a cross piece, or a spur, or an element in the form of a « pig's tall » or else a sphere or a ball, giving to the rod (E) a dumbell configuration and with which each end of this rod (E) is provided, the means with which the two ends of the rod are provided being either identical or different.

7. Device according to claim 1, characterized in that the means preventing any axial movement of the rod (E) is formed by a narrowing (Z) of the diameter of said connection tube (D) at the ends of the rod (E), obtained by molding.

8. Device as claimed in claim 1, characterized in that two auxiliary connection pipes (1), of a diameter less than the diameter of said connection tube (D) containing the rod (E) and formed from the same material as said first and second flexible activating bags (A and B, respectively), connect this tube (D) to these bags (A and B, respectively), thus preventing any axial movement of the rod (E) which it contains.

9. Device as claimed in claim 1, characterized in that said first connecting pipe (D) conencting said third bag (C) to one of said first and second activating bags (A or B) comprises an intermediate gap and a quick sealing junction device (J), particularly clips, reconstituting its physical continuity in the zone of said gap.

10. Prosthetic member formed by an interchangeable urethral prosthesis, comprising a tube made from silicon elastomer, preferably and a means providing sealing of the proximal end of this tube with respect to the bladder (G), which prosthetic member is characterized in that it is provided with a plurality of spurs formed on the external surface of said tube and preventing the axial movement thereof, some spurs being directed downwards, whereas other spurs are directed upwards, and in that it comprises a device for controlling the urinary flow, according to any one of claims 1 to 9.

11. Prosthetic member according to claim 10, characterized in that the length and the diameter of its tube are appropriate to masculine use, and in that said control device is in accordance with claims 1 and 8 or 7 and is included in the wall of the tube of the prosthetic member.

12. Prosthetic member according to claim 10, characterized in that the length and the diameter of its tube are appropriate to masculine use, and in that said control device is in accordance with claims 1 and 6 and 9 and is implantable in an appropriate subcutaneous position.

13. Prosthetic member according to claim 10, characterized in that the length and the diameter of its tube are appropriate to feminine use, and in that said prosthetic tube is provided with a second collar (P'') at its distal end, and in that said control device is in accordance with claims 1, 6 and 9 and is implantable in an appropriate subcutaneous position.

14. Prosthetic member according to claim 10, characterized in that the length and the diameter of its tube are appropriate to feminine use, and in that said prosthetic tube is provided with a second collar (P'') at its distal end, and in that said control device is in accordance with claims 1, 6 and 9 and is in a vaginal position possibly extending beyond the vagina.

15. Prosthetic member according to any one of claims 10 to 14, characterized in that said sealing means with which the proximal end of the prosthetic tube is provided is formed by a collar (p or p') providing the sealing required with respect to the bladder (G).

16. Prosthetic member according to any one of claims 10 to 14, characterized in that said sealing means with which the proximal end of the prosthetic tube is provided is formed by an annular bag which surrounds it, thus providing the sealing required with respect to the bladder (G) in the manner of a Foley probe more particularly.

**Patentansprüche**

1. Strömungsregulierungsvorrichtung, insbesondere für eine biologische Flüssigkeit durch einen anatomischen Kanal und zwar speziell für den Urinfluß mit :

- entweder zwei insbesondere aus einem Silikonelastomer bestehenden, verformbaren Aktivierballons (A, B), die durch einen Verbindungsschlauch (D) miteinander verbunden sind und von denen der erste (A) dazu bestimmt ist, die Wiederherstellung des Flusses der biologischen Flüssigkeit durch Entleeren infolge manueller Deformation zu steuern, während der zweite Aktivierballon (B) ebenfalls durch Entleeren infolge manueller Deformation dazu bestimmt ist, das Sperren dieses Flusses zu steuern ;

- oder mit den beiden Aktivierballons (A, B), die durch den Verbindungsschlauch (D) miteinander verbunden sind, und einem dritten verformbaren Ballon (C), dessen Entleerung und Füllung, die durch manuelle Deformation des ersten und zweiten Aktivierballons (A, B) gesteuert werden, jeweils das Steuern des Strömungsflusses der biologischen Flüssigkeit insbesondere gestatten, indem dieser dritte Ballon (C) in das Lumen des Kanals eingesetzt wird, dessen Strömung man zu steuern wünscht, wobei der dritte Ballon (C) insbesondere durch einen ersten Verbindungstubus (t) geeigneter Länge mit dem ersten Aktivierballon (A) verbunden ist,

- und mit einem Ventilsystem, das ausschließlich auf einen Druck anspricht, der durch manuelle Deformation eines der erwähnten ersten und zweiten Aktivierballons (A, B) erhalten wurde, wobei die Vorrichtung dadurch gekennzeichnet ist, daß das erwähnte Ventilsystem einen Stift (E) aufweist, der in Ruhestellung in dichtender Weise durch jedwede geeignete Befestigungseinrichtung im Innern des erwähnten Verbindungsschlauches (D) eingefaßt ist, die aus einem weichen Kunststoffmaterial hergestellt ist, das beim Polymerisieren ein Schrumpfen aufweist, und daß auch eine Einrichtung vorgesehen ist, die geeignet ist, das axiale Verschieben dieses Ventilstiftes (E) entlang dem Verbindungsschlauch (D) unter der Wirkung des erwähnten Druckes zu verhindern.

2. Vorrichtung nach Anspruch 1, dadurch gekennzeichnet, daß die Befestigungseinrichtung des Stiftes (E) durch einen zweiten Schlauch gebildet ist, der den Verbindungsschlauch (D) umgibt.

3. Vorrichtung nach Anspruch 1, dadurch gekennzeichnet, daß die Befestigungseinrichtung durch einen Ring gebildet ist, der den Befestigungsschlauch (D) umgibt.

4. Vorrichtung nach Anspruch 1, dadurch gekennzeichnet, daß die Befestigungseinrichtung durch eine Feder gebildet ist, die den Befestigungsschlauch (D) umgibt.

5. Vorrichtung nach Anspruch 1, dadurch gekennzeichnet, daß die Befestigungseinrichtung durch die Wandung selbst des Verbindungsschlauches (D) gebildet ist.

6. Vorrichtung nach Anspruch 1, dadurch gekennzeichnet, daß die Einrichtung, die jedwedes axiale Verschieben des Stiftes (E) verhindert, ein Mittel aus der Gruppe ist, die eine Scheibe (r) oder ein Kreuz oder einen Vorsprung oder ein Element in Gestalt eines « Schweineschwanzes » oder auch eine Sphäre oder Kugel umfaßt, das dem Stift (E) eine Hantelgestalt verleiht und das an jedem Ende dieses Stiftes (E) vorgesehen ist, wobei die Mittel, mit denen die beiden Enden des Stiftes versehen sind, identisch oder verschieden sein können.

7. Vorrichtung nach Anspruch 1, dadurch gekennzeichnet, daß das Mittel, das jedwedes axiale Verschieben des Stiftes (E) verhindert, durch durch Formen erhaltene Einschnürungen (z) des Verbindungsschlauches (D) an den den Enden des Stiftes (E) zugeordneten Stellen gebildet ist.

8. Vorrichtung nach Anspruch 1, dadurch gekennzeichnet, daß die beiden Hilfsverbindungstuben (1) mit einem Durchmesser, der kleiner als der Durchmesser des Verbindungsschlauches (D) ist, den Stift (E) enthalten und aus dem gleichen Material hergestellt sind, wie die verformbaren ersten und zweiten Aktivierballons (jeweils A und B), die diesen Schlauch (D) mit diesen Ballons (jeweils A und B) verbinden, wodurch jedwedes axiale Verschieben des in ihm enthaltenen Stiftes (E) verhindert ist.

9. Vorrichtung nach Anspruch 1, dadurch gekennzeichnet, daß erste Verbindungstubus (t), der den dritten Ballon (C) mit einem der ersten und zweiten Aktivierballons (A oder B) verbindet, einen Zwischenschnitt und eine dichte Verbindungseinrichtung (j), insbesondere mit einem Schnappverschluß, aufweist, die seine körperliche Kontinuität in der Zone des erwähnten Schnittes wiederherstellt.

10. Protheseorgan, das aus einer auswechselbaren Harnröhrenprothese besteht, mit einer Hülse vorzugsweise aus einem Silikonelastomer und einer Einrichtung, die die Dichtheit des proximalen Endes dieser Hülse in Bezug auf die Blase (G) gewährleistet, wobei das Protheseorgan dadurch gekennzeichnet ist, daß es mit einer Vielzahl von auf der Außenseite der erwähnten Hülse ausgebildeten und eine axiale Verschiebung der letzteren verhindernden Vorsprüngen versehen ist, wobei gewisse Vorsprünge nach unten gerichtet sind, während andere Vorsprünge nach oben gerichtet sind, und daß es eine Vorrichtung zur Steuerung des Urinflusses gemäß einem der Ansprüche 1 bis 9 aufweist.

11. Protheseorgan nach Anspruch 10, dadurch gekennzeichnet, daß die Länge und der Durchmesser seiner Hülse für die Verwendung bei einem männlichen Patienten angepaßt sind und daß die Steuereinrichtung gemäß den Ansprüchen 1 und 8 oder 7 ausgebildet ist und in der Wandung der Hülse des Protheseorganes enthalten ist.

12. Protheseorgan nach Anspruch 10, dadurch gekennzeichnet, daß die Länge und der Durchmesser seiner Hülse für die Verwendung bei einem männlichen Patienten geeignet sind und daß die Steuereinrichtung gemäß den Ansprüchen 1, 6 und 9 ausgebildet und an einer geeigneten subkutanen Stelle implantierbar ist.

13. Protheseorgan nach Anspruch 10, dadurch gekennzeichnet, daß die Länge und der Durchmesser seiner Hülse für die Verwendung bei einem weiblichen Patienten geeignet sind, daß die prothetische Hülse mit einem zweiten Kragen (p'') an ihrem distalen Ende versehen ist und daß die Steuervorrichtung entsprechend den Ansprüchen 1, 6 und 9 ausgebildet und an einer geeigneten Stelle subkutan implantierbar ist.

14. Protheseorgan nach Anspruch 10, dadurch gekennzeichnet, daß die Länge und der Durchmesser seiner Hülse für die Verwendung bei einem weiblichen Patienten geeignet sind, daß die prothetische Hülse mit einem zweiten Kragen (p'') an ihrem distalen Ende versehen ist und daß die erwähnte Steuervorrichtung entsprechend den Ansprüchen 1, 6 und 9 ausgebildet und in einer vaginalen Position angeordnet ist, die gegebenenfalls aus der Vagina hinausragt.

15. Protheseorgan nach einem der Ansprüche 10 bis 14, dadurch gekennzeichnet, daß die erwähnte Dichtungseinrichtung, mit der das proximale Ende der prothetischen Hülse versehen ist, durch einen Kragen (p oder p') gebildet ist, der die notwendige Dichtheit gegenüber der Blase (G) gewährleistet.

16. Protheseorgan nach einem der Ansprüche 10 bis 14, dadurch gekennzeichnet, daß die Dichtungseinrichtung, mit der das proximale Ende der prothetischen Hülse versehen ist, durch einen sie umgebenden Ringballon gebildet ist, der auf diese Weise die notwendige Abdichtung gegenüber der Blase (G), insbesondere in der Weise einer Foley-Sonde gewährleistet.

FIG.1a

FIG.1b

FIG. 2

FIG.1c

FIG. 3

FIG. 5

FIG. 4

I

II